# EUROPÄISCHE PATENTSCHRIFT

(11) **EP 1 753 711 B1**
(45) Veröffentlichungstag und Bekanntmachung des Hinweises auf die Patenterteilung: **08.07.2015**
(21) Anmeldenummer: 05748278.8
(22) Anmeldetag: 27.05.2005
(51) Int. Cl.: C07C 209/22, C07C 213/08, C07D 295/023, C07D 295/037

(54) **VERFAHREN ZUR HERSTELLUNG VON QUARTÄREN AMMONIUM-VERBINDUNGEN**
METHOD FOR PRODUCING QUATERNARY AMMONIUM COMPOUNDS
PROCÉDÉ DE PRODUCTION DE COMPOS S D'AMMONIUM QUATERNAIRES

(30) Priorität: 28.05.2004 DE 102004026153; 07.02.2005 DE 102005005582
(43) Veröffentlichungstag der Anmeldung: 21.02.2007
(73) Patentinhaber: BASF SE, 67056 Ludwigshafen (DE)
(72) Erfinder: SZARVAS, Laszlo, 67071 Ludwigshafen (DE); MASSONNE, Klemens, 67098 Bad Dürkheim (DE); MAASE, Matthias, 67346 Speyer (DE); OFTRING, Alfred, 67098 Bad Dürkheim (DE)
(86) Internationale Anmeldenummer: PCT/EP2005/005727
(87) Internationale Veröffentlichungsnummer: WO 2005/115969

(56) Entgegenhaltungen:
- DE-A1- 3 705 896
- GB-A- 823 595
- US-A- 3 366 663
- US-A- 4 572 769
- PATENT ABSTRACTS OF JAPAN Bd. 006, Nr. 223 (C-133), 9. November 1982 (1982-11-09) & JP 57 126465 A (ASAHI KASEI KOGYO KK), 6. August 1982 (1982-08-06)

## Beschreibung

Die vorliegende Erfindung betrifft ein Verfahren zur Herstellung von quartären Ammonium-Verbindungen, bei dem man Verbindungen, die ein sp³-hybridisiertes Stickstoffatom enthalten, mit einem Dialkylsulfat oder Trialkylphosphat umsetzt und die so erhaltene Ammonium-Verbindung einem Anionenaustausch unterzieht.

Quartäre Ammonium-Verbindungen werden in großen Mengen für diverse Einsatzbereiche verwendet. So besitzen quartäre Ammonium-Verbindungen mit mindestens einer langen Alkylkette oberflächenaktive Eigenschaften und werden als Kationtenside z. B. als Netzmittel, Antistatika, etc. eingesetzt. Vorwiegend kurzkettige quartäre Ammonium-Verbindungen weisen mikrobizide Eigenschaften auf und finden daher Verwendung in fungiziden und bakteriziden Desinfektionsmitteln. In der organischen Synthese werden quartäre Ammonium-Verbindungen als Phasentransferkatalysatoren eingesetzt. Zudem gibt es diverse technische Einsatzgebiete für einzelne spezielle quartäre Ammonium-Verbindungen. Bei niedrigen Temperaturen (< 100 °C) flüssige Salze aus quartären Ammonium-Ionen und geeigneten Anionen haben inzwischen breite Verwendung als so genannte ionische Flüssigkeiten (ionic liquids) gefunden.

Die zunehmend fortschreitende Verkleinerung elektronischer Bauteile erfordert die Bereitstellung immer leistungsfähiger Mikrochips mit immer höheren Integrationsgraden. Diese lassen sich nur aus hochreinen Silicium-Wafern herstellen, deren Gehalt an Verunreinigungen im Allgemeinen in einem Bereich von höchstens 10 ppb und teilweise weit darunter liegt. Bei der Herstellung solcher elektronischer Bauteile treten Verfahrensschritte auf, wie z. B. das Ätzen und Reinigen, bei dem die Halbleiter mit verschiedenen Chemikalien behandelt werden. Dabei wird beispielsweise hochreines Tetramethylammoniumhydroxid zum Ätzen der Rückseiten von Siliciumwafern und als Entwickler für Fotolacke eingesetzt.

Ein wesentliches Problem bei der Herstellung hochintegrierter Schaltkreise ist eine Verunreinigung der Halbleiteroberflächen durch anorganische oder organische Verbindungen, die in den eingesetzten Chemikalien enthalten sind. Daher werden an aile Reagenzien, die in Kontakt mit Halbleiterelementen kommen, extrem hohe Reinheitsanforderungen gestellt, z. B. soll der Gehalt von ionischen Verunreinigungen möglichst niedrig sein. Bevorzugt sollen diese Lösungen einen Gesamtmetallgehalt von höchstens 5 ppb und zum Teil,weniger als 10 ppt für einzelne Metallspezies besitzen. Auch eine Verunreinigung mit bestimmten Anionen muss vermieden werden, da diese entweder korrosiv wirken oder Metall-Ionen komplexieren und damit die gezielte Dotierung des Halbleiters verändern können. Der Gesamtgehalt an solchen Anionen soll in der Regel 1 ppm nicht übersteigen.

Aus diesem Grunde besteht ein großer Bedarf an hochreinem Tetramethylammoniumhydroxid-Lösungen, die im Wesentlichen frei von unerwünschten organischen Verbindungen sind.

Verfahren zur Abtrennung unerwünschter organischer Verunreinigungen aus Lösungen quartärer Ammonium-Verbindungen, wie Tetramethylammoniumhydroxid-Lösungen, sind bekannt, in der Regel jedoch aufwendig und entsprechend kostspielig. Es besteht somit ein großer Bedarf an Verfahren, die sich zur wirtschaftlichen Herstellung von quartären Ammonium-Verbindungen eignen, die im Wesentlichen frei von unerwünschten organischen Verunreinigungen sind.

Es ist bekannt, dass sich Amine mit Dialkylsulfaten alkylieren lassen, wobei in der Regel jedoch nur eine Alkylgruppe des Dialkylsulfats ausgenutzt wird, so dass die entsprechenden Monoalkylsulfatsalze resultieren. So beschreibt die US 3,366,663 ein Verfahren zur Herstellung von Tetraalkylammoniumalkylsulfaten, bei dem man ein Dialkylsulfat, z. B. Dimethylsulfat, mit einem Trialkylamin umsetzt.

Die EP-A-1 182 196 beschreibt ein Verfahren zur Herstellung ionischer Flüssigkeiten, bei dem man die dem Kation zu Grunde liegenden Amine, Phosphine, Imidazole, Pyridine, Triazole oder Pyrazole mit einem Dialkylsulfat.alkyliert, wobei Salze der entsprechenden Monoalkylsulfatanionen erhalten werden und man diese anschließend einem Anionenaustausch mit Metallsalzen unterzieht.

Die WO 02/12179 beschreibt ein Verfahren zur Sulfatierung von Verbindungen mit Hydroxylgruppen. Dabei wird als Sulfatierungsmittel ein aus einem tertiären Amin und einem Diorganylsulfat gebildetes Ammoniummonoorganylsulfat eingesetzt.

J. S. Wilkes und M. J. Zaworotko beschreiben in J. Chem. Soc., Chem. Commun., 1992, S. 965 - 967 ionische Flüssigkeiten auf Basis des 1-Ethyl-3-methylimidazolium-Kations. Ausgehend von der Iodidverbindung lassen sich weitere Anionen, z. B. das Sulfat in Form seines Monohydrats, durch Anionenaustausch mit den entsprechenden Silbersalzen herstellen.

Die WO 03/074494 beschreibt halogenfreie ionische Flüssigkeiten auf Basis von Anionen der Formeln [R'-O-SO₃]⁻ oder [R'-SO₃]⁻, wobei R' eine Gruppe der allgemeinen Formel R⁵-[X(-CH₂-)ₙ]ₘ darstellt, in der n eine Zahl zwischen 1 und 12 ist, m eine Zahl zwischen 1 und 400 ist, X für Sauerstoff, Schwefel oder eine Gruppe der allgemeinen Formeln -O-Si(CH₃)₂-O-, -O-Si(CH₂CH₃)₂-O-, -O-Si(OCH₃)₂-O- oder -O-Si(O-CH₂CH₃)₂-O- steht und R⁵ eine gegebenenfalls funktionalisierte Alkylgruppe darstellt. Ihre Herstellung erfolgt ausgehend von Pyridin-SO₃-Komplexen und Ethern der Formel R'-OH.

Das belgische Patent BE 750 372 beschreibt ein Verfahren zur Herstellung neutraler quaternärer Ammoniumsalze polybasischer Säuren, bei dem man ein quaternäres Ammoniumsalz eines sauren Esters einer polybasischen Säure, z. B. ein Tetraalkylammoniumalkylsulfat, hydrolysiert und anschließend mit einem Alkalihydroxid behandelt.

Die JP-A- 57 126465 beschreibt ein Verfahren zur Herstellung von Tetraalkylammoniumsalzen, bei dem man ein Tetraalkylammoniumalkylsulfat, z. B. Tetraethylammoniumethylsulfat, mit einem Anionenaustauscher mit OH⁻-Anionen behandelt und das resultierende Tetraalkylammoniumhydroxid mit einer Säure neutralisiert.

Die DE-OS-15 43 747 (US 3,371,117) beschreibt ein Verfahren zur direkten Herstellung eines bisquaternären Ammoniumsalzes aus einem Dialkylsulfatester und einem Trialkylamin durch Umsetzung bei einer Temperatur im Bereich von 0 bis 400 °C und einem ausreichenden Druck, um die Verdampfung des Amins zu verhindern. Da bei erhöhten Temperaturen eine Hydrolyse des Sulfatesters stattfindet, lehrt dieses Dokument die Umsetzung zweistufig durchzuführen, wobei zunächst bei einer niedrigen Temperatur im Bereich von etwa 0 bis 50 °C eine Alkylgruppe des Sulfatesters und dann in einem zweiten Schritt bei einer erhöhten Temperatur im Bereich von etwa 50 bis 400 °C die zweite Alkylgruppe zur Alkylierung eingesetzt wird.

Die unveröffentlichte deutsche Patentanmeldung 10 2004 010 662.2 beschreibt ein Verfahren zur Herstellung von ionischen Verbindungen umfassend Kationen mit quaternären sp²-hybridisierten Stickstoffatomen, bei dem man Verbindungen, die ein doppelt gebundenes Stickstoffatom enthalten, mit einem Dialkylsulfat bei erhöhter Temperatur und unter Einsatz beider Alkylgruppen des Dialkylsulfats umsetzt und die so erhaltene ionische Verbindung mit Sulfatanionen gegebenenfalls einem Anionenaustausch unterzieht.

Der vorliegenden Erfindung liegt die Aufgabe zu Grunde, ein einfaches und somit wirtschaftliches Verfahren zur Herstellung von quartären Ammonium-Verbindungen zur Verfügung zu stellen. Insbesondere soll sich das Verfahren zur Herstellung von hochreinen quartären Ammonium-Verbindungen, wie Tetramethylammoniumhydroxid, eignen, die im Wesentlichen frei von unerwünschten organischen Verunreinigungen sind. Speziell sollen die so erhaltenen quartären Ammonium-Verbindungen frei von Halogeniden, speziell frei von Chlorid, Bromid und Iodid, sein.

Überraschenderweise wurde nun gefunden, dass diese Aufgabe durch ein Verfahren gelöst wird, bei dem man eine Amin-Verbindung, die wenigstens ein sp³-hybridisiertes Stickstoffatom enthält, mit einem Dialkylsulfat umsetzt, wobei eine quartäre Ammoniumverbindung erhalten wird, die als Anionkomponente ein Sulfatanion enthält, und anschließend das Sulfatanion gegen ein davon verschiedenes Anion austauscht.

Gegenstand der Erfindung ist daher ein Verfahren zur Herstellung einer quartären Ammonium-Verbindung, bei dem man
a) eine Amin-Verbindung, die wenigstens ein sp³-hybridisiertes Stickstoffatom enthält, mit einem Dialkylsulfat oder Trialkylphosphat unter Erhalt einer quartären Ammoniumverbindung umsetzt, die zumindest teilweise mehrwertige Anionen aufweist, und
b) die in Schritt a) erhaltene quartäre Ammoniumverbindung einem Anionenaustausch unterzieht.

Überraschenderweise wurde gefunden, dass sich Amin-Verbindungen, die wenigstens ein sp³-hybridisiertes Stickstoffatom aufweisen, mit Dialkylsulfaten oder Trialkylphosphaten vorteilhaft quaternisieren lassen. Dabei werden quartäre Ammoniumverbindung erhalten, die zumindest teilweise, vorzugsweise ausschließlich, mehrwertige Anionen aufweisen. In einer ersten bevorzugten Ausführung dieses Verfahrens wird in Schritt a) die Amin-Verbindung mit einem Dialkylsulfat unter Einsatz beider Alkylgruppen des Dialkylsulfats umgesetzt, wobei eine quartäre Ammoniumverbindung mit Sulfatanionen (SO₄²⁻) erhalten wird. In einer zweiten bevorzugten Ausführung dieses Verfahrens wird in Schritt a) die Amin-Verbindung mit einem Trialkylphosphat unter Einsatz zweier oder dreier Alkylgruppen des Trialkylphosphats umgesetzt, wobei eine quartäre Ammoniumverbindung mit Phosphatanionen (PO₄³⁻) und/oder Monoalkylphoshatanionen (RPO₄²⁻ R=Alkyl) erhalten wird.

Vorteilhafterweise werden somit quartäre Ammonium-Verbindungen erhalten, die als Anionenkomponente zweifach und/oder dreifach negativ geladene Anionen anstelle von einfach negativ geladenen Anionen (Monoalkylsulfat, Dialkylphosphat) aufweisen. Somit können zum einen die Alkylgruppenäquivalente des Dialkylsulfats oder Trialkylphosphats effektiv ausgenutzt werden, zum anderen sind die erhaltenen Verbindungen mit mehrwertigen Anionen gute Zwischenprodukte für die Herstellung quartärer Ammonium-Verbindungen, die frei von unerwünschten Verunreinigungen sind, insbesondere halogenidfreie quartäre Ammonium-Verbindungen. Vorteilhafterweise wird die im Stand der Technik als Nachteil der zweifachen Alkylierung mit Dialkylsulfaten beschriebene Hydrolyse beim erfindungsgemäßen Verfahren nicht beobachtet. Der Einsatz aufwendiger Reinigungsschritte kann somit in der Regel vermieden werden.

Für den Zweck der Erläuterung der vorliegenden Erfindung umfasst der Ausdruck "Alkyl" geradkettige und verzweigte Alkylgruppen. Vorzugsweise handelt es sich dabei um geradkettige oder verzweigte C₁-C₂₀-Alkyl, bevorzugterweise C₁-C₁₀-Alkyl-, besonders bevorzugt C₁-C₈-Alkyl- und ganz besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Ausdruck "Alkyl" umfasst auch substituierte Alkylgruppen, welche im Allgemeinen 1, 2, 3, 4 oder 5, bevorzugt 1, 2 oder 3 und besonders bevorzugt 1 Substituenten aufweisen. Diese sind beispielsweise ausgewählt unter Cycloalkyl, Aryl, Hetaryl, Halogen, Hydroxy, Mercapto (Sulfhydryl, Thiol), Amino, Alkoxycarbonyl, Acyl, Nitro, Aminocarbonyl, Alkylaminocarbonyl, Dialkylaminocarbonyl, Alkylcarbonylamino, Carboxylat, Thioester und Sulfonat.

Der Ausdruck "Alkylen" im Sinne der vorliegenden Erfindung steht für geradkettige oder verzweigte Alkandiyl-Gruppen mit vorzugsweise 1 bis 5 Kohlenstoffatomen.

Der Ausdruck "Cycloalkyl" Umfasst im Sinne der vorliegenden Erfindung unsubstituierte als auch substituierte Cycloalkylgruppen, vorzugsweise C₅-C₈-Cycloalkylgruppen, wie Cyclopentyl, Cyclohexyl oder Cycloheptyl. Diese können im Falle einer Substitution, im Allgemeinen 1, 2, 3, 4 oder 5; bevorzugt 1, 2 oder 3 Substituenten tragen. Diese Substituenten sind beispielsweise ausgewählt unter Alkyl und den zuvor für substituierte Alkylgruppen genannten Substituenten.

Der Ausdruck "Heterocycloalkyl" im Sinne der vorliegenden Erfindung umfasst gesättigte, cycloaliphatische Gruppen mit im Allgemeinen 4 bis 7, vorzugsweise 5 oder 6 Ringatomen, in denen 1, 2, 3 oder 4 der Ringkohlenstoffatome durch Heteroatome, ausgewählt aus den Elementen Sauerstoff, Stickstoff und Schwefel, ersetzt sind und die gegebenenfalls substituiert sein können. Im Falle einer Substitution können diese heterocycloaliphatischen Gruppen z. B. 1, 2 oder 3 Substituenten tragen. Diese Substituenten sind beispielsweise ausgewählt unter Alkyl und den zuvor für substituierte Alkylgruppen genannten Substituenten. Beispielhaft für solche heterocycloaliphatischen Gruppen seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl, Tetrahydrothiophenyl, Tetrahydrofuranyl, Tetrahydropyranyl, Dioxanyl genannt.

Der Ausdruck "Aryl" umfasst im Sinne der vorliegenden Erfindung unsubstituierte als auch substituierte Arylgruppen, und steht vorzugsweise für Phenyl, Tolyl, Xylyl, Mesityl, Naphthyl, Fluorenyl, Anthracenyl, Phenanthrenyl oder Naphthacenyl, besonders bevorzugt für Phenyl oder Naphthyl. Diese Arylgruppen können im Falle einer Substitution im Allgemeinen 1, 2, 3, 4 oder 5, vorzugsweise 1, 2 oder 3 Substituenten tragen. Diese Substituenten sind beispielsweise ausgewählt unter Alkyl und den zuvor für substituierte Alkylgruppen genannten Substituenten.

Der Ausdruck "Hetaryl" umfasst im Sinne der vorliegenden Erfindung unsubstituierte oder substituierte, heterocycloaromatische Gruppen, vorzugsweise die Gruppen Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl. Diese heterocycloaromatischen Gruppen können im Falle einer Substitution im Allgemeinen 1, 2 oder 3 Substituenten. Diese Substituenten sind beispielsweise ausgewählt unter Alkyl und den zuvor für substituierte Alkylgruppen genannten Substituenten.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäureester- oder Sulfonsäureesterfunktion oder eine Carbonsäure-oder Sulfonsäureamidfunktion. Dazu zählen z. B. die Ester mit C₁-C₄-Alkanolen, wie Methanol, Ethanol, n-Propanol, Isopropanol, n-Butanol, sec.-Butanol und tert.-Butanol.

Die obigen Erläuterungen zu den Ausdrücken "Alkyl", "Cycloalkyl", "Aryl", "Heterocycloalkyl" und "Hetaryl" gelten entsprechend für die Ausdrücke "Alkoxy", "Cycloalkoxy", "Aryloxy", "Heterocycloalkoxy" und "Hetaryloxy".

Der Ausdruck "Acyl" steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Gruppen NE¹E²stehen vorzugsweise für N,N-Dimethylamino, N,N-Diethylamino, N,N-Dipropylamino, N,N-Diisopropylamino, N,N-Di-n-butylamino, N,N-Di-t.-butylamino, N,N-Dicyclohexylamino oder N,N-Diphenylamino.

Halogen steht für Fluor, Chlor, Brom und Iod, bevorzugt für Fluor, Chlor und Brom.

M⁺ steht für ein Kationäquivalent, d. h. für ein einwertiges Kation oder den einer positiven Einfachladung entsprechenden Anteil eines mehrwertigen Kations. Das Kation M⁺ dient lediglich als Gegenion zur Neutralisation negativ geladener Substituentengruppen, wie der COO- oder der Sulfonatgruppe und kann im Prinzip beliebig gewählt werden. Vorzugsweise werden deshalb Alkalimetall-, insbesondere Na⁺, K⁺-, Li⁺-Ionen oder Onium-Ionen, wie Ammonium-, Mono-, Di-, Tri-, Tetraalkylammonium-, Phosphonium-, Tetraalkylphosphonium- oder Tetraarylphosphonium-Ionen verwendet.

Entsprechendes gilt für das Anionäquivalent A⁻, das lediglich als Gegenion positiv geladener Substituentengruppen, wie den Ammoniumgruppen, dient und beliebig gewählt werden kann unter einwertigen Anionen und den einer negativen Einfachladung entsprechenden Anteilen eines mehrwertigen Anions, wobei im Allgemeinen von Halogenid-Ionen verschiedene Anionen bevorzugt sind.

Der Begriff polycyclische Verbindungen umfasst im Rahmen der vorliegenden Erfindung im weitesten Sinn Verbindungen, die wenigstens zwei Ringe enthalten, unabhängig davon, wie diese Ringe verknüpft sind. Hierbei kann es sich um carbocyclische und/oder heterocyclische Ringe handeln. Die Ringe können über Einfach- oder Doppelbindung verknüpft ("mehrkernige Verbindungen"), durch Anellierung verbunden ("kondensierte Ringsysteme") oder überbrückt ("überbrückte Ringsysteme", "Käfigverbindungen") sein. Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach derVerknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört. Zu den überbrückten Ringsystemen zählen im Rahmen der vorliegenden Erfindung solche, die nicht zu den mehrkernigen Ringsystemen und nicht zu den kondensierten Ringsystemen zählen und bei denen mindestens zwei Ringatome zumindest zwei verschiedenen Ringen angehören. Bei den überbrückten Ringsystemen unterscheidet man je nach Anzahl der Ringöffnungsreaktionen, die formal erforderlich sind, um zu einer offenkettigen Verbindung zu gelangen, Bi-, Tri-, Tetracyclo-Verbindungen usw., die aus zwei, drei, vier usw. Ringen bestehen. Die überbrückten Ringsysteme können gewünschtenfalls zusätzlich, je nach Größe, einen, zwei, drei oder mehr als drei ankondensierte Ringe aufweisen.

Das erfindungsgemäße Verfahren eignet sich ganz allgemein zur Herstellung von ionischen Verbindungen der Formel I

b B^{m+} x Xⁿ⁻ (I)

worin
- B^{m+}: für ein m-wertiges Kation mit wenigstens einem quaternären sp³-hybridisierten Stickstoffatom steht,
- Xⁿ⁻: für ein n-wertiges Anion steht,
b und x für ganze Zahlen ≥ 1 stehen, mit der Maßgabe, das (b mal m) = (x mal n) ist.

Dazu zählen Verbindungen der Formeln B⁺ X⁻, B^{m+} X^{m-}, nB⁺ Xⁿ⁻ und B^{m+} mX⁻, worin m und n für ganze Zahlen > 1 stehen.

Vorzugsweise handelt es sich bei der Anionkomponente Xⁿ⁻ um ein von Cl⁻, Br⁻, I⁻, Monoalkylsulfaten und Monoalkylphosphaten verschiedenes Anion. Vorzugsweise sind die Anionen Xⁿ⁻ ausgewählt unter Hydroxid OH⁻, Sulfat (SO₄²⁻), Hydrogensulfat (HSO₄⁻), Nitrit (NO₂⁻), Nitrat (NO₃⁻), Cyanid (CN⁻), Cyanat (OCN⁻), Isocyanat (NCO⁻), Thiocyanat (SCN⁻), Isothiocyanat (NCS⁻), Phosphat (PO₄³⁻), Hydrogenphosphat (HPO₄²⁻), Dihydrogenphosphat (H₂PO₄⁻), primärem Phosphit (H₂PO₃⁻), sekundärem Phosphit (HPO₃²⁻), Orthoborat (BO₃³⁻), Metaborat ((BO₂)₃³⁻), Pentaborat B₅O₈⁻, Pentaborat-Hydrat (B₅H₄O₁₀⁻), B₅O₆⁻, Tetrafluoroborat ([BF₄]⁻), Tetrachloroborat ([BCl₄]⁻), Tetraphenylborat ([B(C₆H₅)₄]⁻), Hexafluorophosphat ([PF₆]⁻), Hexafluoroantimonat ([SbF₆]⁻), Hexafluoroarsenat ([AsF₆]⁻), Tetrachloroaluminat ([AlCl₄]⁻), Tetrachbromoaluminat ([AlBr₄]⁻), Trichlorozinkat ([ZnCl₃]⁻), Dichlorocupraten(I) und (II), Carbonat (CO₃²⁻), Hydrogencarbonat (HCO₃⁻), Fluorid (F⁻), Triorganylsilanolat (R₃SiO⁻), Fluorosulfonat (CF₃-SO₃⁻), Sulfonat (R'-SO₃)⁻ und [(R'-SO₂)₂N]⁻, worin R' für Alkyl, Cycloalkyl oder Aryl steht. Bevorzugt ist R' ein linearer oder verzweigter 1 bis 12 Kohlenstoffatome enthaltender aliphatischer oder alicyclischer Alkyl- oder ein C₅-C₁₈-Aryl-, C₅C₁₈-Aryl-C₁-C₆-alkyl- oder C₁-C₆-Alkyl-C₅-C₁₈-aryl-Rest, der durch Halogenatome substituiert sein kann.

Besonders bevorzugt steht Xⁿ⁻ für OH⁻.

Bei der in Schritt a) eingesetzten Amin-Verbindung, die wenigstens ein sp³-hybridisiertes Stickstoffatom enthält kann es sich um eine acyclische oder cyclische Verbindung handeln. Von diesen Aminen leitet sich die Kationkomponente B^{m+} durch Quaternisierung ab.

Geeignete Amin-Verbindungen weisen wenigstens eine primäre, sekundäre oder tertiäre Aminofunktion auf. Sie sind vorzugsweise ausgewählt unter Verbindungen der allgemeinen Formel NR¹R²R³, worin R¹ R² und R³ unabhängig voneinander ausgewählt sind unter Wasserstoff, Alkyl, Cycloalkyl, Heterocycloalkyl, Aryl und Hetaryl, wobei wenigstens zwei der Reste R¹ R² und R³ gemeinsam mit dem N-Atom, an das sie gebunden sind, auch Teil einer polycyclischen Verbindung sein können.

Vorzugsweise sind die Reste R¹, R² und R³ unabhängig voneinander ausgewählt unter Wasserstoff, C₁-C₃₀-Alkyl-, C₃-C₈-Cycloalkyl-, C₃-C₈-Heterocycloalkyl-, C₁-C₁₄-Aryl und C₁-C₁₄-Heteroarylresten.

Wenn wenigstens einer der Reste R¹ bis R³ für Alkyl steht, so handelt es sich vorzugsweise um C₁-C₂₀-Alkylreste, die, wie eingangs definiert, substituiert und/oder durch 1, 2, 3, oder mehr als 3 nicht benachbarte Heteroatome oder heteroatomhaltige Gruppen unterbrochen sein können. Die Heteroatome und heteroatomhaltigen Gruppen sind dabei vorzugsweise ausgewählt unter O, S, NR⁴ oder PR⁵, worin R⁴ und R⁵ unabhängig voneinander für Wasserstoff, Alkyl, Cycloalkyl, Heterocycloalkyl, Aryl, Hetaryl, COOR^{a}, COO-M⁺, SO₃R^{a}, SO₃⁻M⁺, Sulfonamid, NE¹E², (NE¹E²E³)⁺A⁻, OR^{a}, SR^{a}, (CHR^{b}CH₂O)_{y}R^{a}, (CH₂O)_{y}R^{a}, (CH₂CH₂NE¹)_{y}R^{a}, Alkylaminocarbonyl, Dialkylaminocarbonyl, Alkylcarbonylamino, Halogen, Nitro, Acyl oder Cyano stehen, worin
- R^{a}: jeweils gleiche oder verschiedene Reste, ausgewählt unter Wasserstoff, Alkyl, Cycloalkyl, Aryl, Heterocycloalkyl oder Hetaryl bedeuten,
- E¹, E², E³: jeweils gleiche oder verschiedene Reste, ausgewählt unter Wasserstoff, Alkyl, Cycloalkyl, Aryl oder Hetaryl bedeuten,
- R^{b}: für Wasserstoff, Methyl oder Ethyl steht,
- M⁺: für ein Kationäquivalent steht,
- A⁻: für ein Anionäquivalent steht und
- y: für eine ganze Zahl von 1 bis 250 steht.

Wenn wenigstens einer der Reste R¹ bis R³ für substituiertes Alkyl steht, so sind die Substituenten vorzugsweise ausgewählt unter Hydroxy, Mercapto, Ester und Thioester.

Geeignete Reste R¹ bis R³ sind beispielsweise Wasserstoff, Methyl, Ethyl, n-Propyl, sek.-Propyl, Isopropyl, n-Butyl, iso-Butyl, sek.-Butyl, tert.-Butyl, n-Pentyl und n-Hexyl, Lauryl, Tridecyl, Myristyl, Palmityl und Stearyl. Geeignete Reste R¹ bis R³ sind weiterhin 5-, 6- und 7-gliedrige gesättigte, ungesättigte oder aromatischen Carbo- und Heterocyclen, wie Cyclopentyl, Cyclohexyl, Phenyl, Toloyl, Xylyl, Cycloheptanyl, Naphthyl, Tetrahydrofuranyl, Tetrahydropyranyl, Dioxanyl, Pyrrolidyl, Piperidyl, Pyridyl und Pyrimidyl.

Geeignete Amin-Verbindungen, die eine primäre Aminofunktion aufweisen, sind beispielsweise Methylamin, Ethylamin, n-Propylamin, Isopropylamin, n-Butylamin, isoButylamin, sek.-Butylamin, tert.-Butylamin, Pentylamin, Hexylamin, Cyclopentylamin, Cyclohexylamin, Anilin und Benzylamin.

Geeignete Amin-Verbindungen, die eine primäre Aminofunktion aufweisen und bei denen einer der Reste R¹ bis R³ für einen durch O unterbrochenen Alkylrest steht sind beispielweise CH₃-O-C₂H₄-NH₂, C₂H₅-O-C₂H₄-NH₂, CH₃-O-C₃H₆-NH₂, C₂H₅-O-C₃H₆-NH₂, n-C₄H₉-O-C₄H₈-NH₂, HO-C₂H₄-NH₂, HO-C₃H₇-NH₂ und HO-C₄H₈-NH₂.

Geeignete Amin-Verbindungen, die eine sekundäre Aminofunktion aufweisen, sind beispielweise Dimethylamin, Diethylamin, Methylethylamin, Di-n-propylamin, Diisopropylamin, Diisobutylamin, Di-sek.-butylamin, Di-tert.-butylamin, Dipentylamin, Dihexylamin, Dicyclopentylamin, Dicyclohexylamin und Diphenylamin.

Geeignete Amin-Verbindungen, die eine sekundäre Aminofunktion aufweisen und bei denen einer oder zwei der Reste R¹ bis R³ für einen durch O unterbrochenen Alkylrest stehen sind beispielweise (CH₃-O-C₂H₄)₂NH, (C₂H₅-O-C₂H₄)₂NH, (CH₃-O-C₃H₆)₂NH, (C₂H₅-O-C₃H₆)₂NH, (n-C₄H₉-O-C₄H₈)₂NH, (HO-C₂H₄)₂NH, (HO-C₃H₆)₂NH und (HO-C4H₈)₂NH.

Geeignete Amin-Verbindungen, die eine tertiäre Aminofunktion aufweisen, sind beispielsweise Trimethylamin, Triethylamin, Tri-(n-propyl)amin, Tri-(isopropyl)amin, Tri-(n-butyl)amin, Tri-(isobutyl)amin Tri-(tert.-butyl)amin etc.

Geeignete Amin-Verbindungen, die eine tertiäre Aminofunktion aufweisen, sind weiterhin Dialkylarylamine, bevorzugt Di-(C₁-C₄-)alkylarylaminen, wobei die Alkylgruppen und/oder die Arylgruppe zusätzlich substituiert sein können. Die Arylgruppe steht vorzugsweise für Phenyl. Dazu zählen z. B. N,N-Dimethylanilin, N,N-Diethylanilin, N,N,2,4,6-Pentamethylanilin, Bis(4-(N,N-Dimethylamino)phenyl)methylen, 4,4'-Bis(N,N-dimethylamino)benzophenon etc.

Geeignete Amin-Verbindungen, die eine tertiäre Aminofunktion aufweisen, sind weiterhin Alkyldiarylamine, bevorzugt (C₁-C₄-)Alkyldiarylaminen, wobei die Alkylgruppe und/oder die Arylgruppen gegebenenfalls substituiert sein können. Dazu zählen z. B. Diphenylmethylamin und Diphenylethylamin.

Geeignete Amin-Verbindungen, die eine tertiäre Aminofunktion aufweisen, sind weiterhin Triarylamine, wobei die Arylgruppen gegebenenfalls substituiert sein können, wie Triphenylamin, etc. Des Weiteren bevorzugte Amine sind Tricycloalkylamine, wie Tricyclohexylamin.

Wenn wenigstens zwei der Reste R¹, R² und R³ gemeinsam mit dem N-Atom, an das sie gebunden sind, Teil einer polycyclischen Verbindung sind, so bilden vorzugsweise zwei der Reste R¹, R² und R³ gemeinsam mit dem N-Atom, an das sie gebunden sind, einen gegebenenfalls substituierten 5- bis 7-gliedrigen Heterocyclus, der ein, zwei oder drei weitere Heteroatome oder heteroatomhaltige Gruppe, ausgewählt unter O, S, NR⁴ oder PR⁵ aufweisen kann, worin R⁴ und R⁵ die zuvor angegebenen Bedeutungen besitzen. Geeignete cyclische Amin-Verbindungen sind beispielsweise Pyrrolidin, Piperidin, Morpholin und Piperazin sowie deren substituierte Derivate, Geeignete Derivate der zuvor genannten stickstoffhaltigen Heterocyclen können z. B. einen oder mehrere C₁-C₆-Alkylsubstituenten, wie Methyl, Ethyl, n-Propyl, Isopropyl, n-Butyl, sec.-Butyl, tert.-Butyl, etc., aufweisen. Dazu zählen beispielsweise die N-C₁- C₆-Alkylderivate.

Des Weiteren bevorzugt bilden die Reste R¹, R² und R³ gemeinsam mit dem N-Atom, an das sie gebunden sind ein bicyclisches Trialkylenamin oder Trialkylendiamin, wie 1-Azabicyclo[2.2.2]octan oder 1,4-Diazabicyclo[2.2.2]octan.

Geeignete Amin-Verbindungen sind weiterhin Alkylendiamine, Dialkylentriamine, Trialkylentetramine und Polyalkylenpolyamine, wie Oligo- oder Polyalkylenimine, insbesondere Oligo- oder Polyethylenimine, bevorzugt Oligoethylenimine, bestehend aus 2 bis 20, vorzugsweise 2 bis 10 und besonders bevorzugt 2 bis 6 Ethylenimineinheiten. Geeignete solche Verbindungen sind insbesondere n-Propylendiamin, 1,4-Butandiamin, 1,6-Hexandiamin, Diethylentriamin, Triethylentetramin und Polyethylenimine, sowie deren Alkylierungsprodukte, die wenigstens eine primäre oder sekundäre Aminofunktion aufweisen, z. B. 3-(Dimethylamino)-n-propylamin, N,N-Dimethylethylendiamin,
N,N-Diethylethylendiamin und N,N,N',N'-Tetramethyldiethylentriamin. Ebenfalls geeignet ist Ethylendiamin.
Weitere geeignete Amin-Verbindungen sind die Umsetzungsprodukte von Alkylenoxiden, insbesondere Ethylenoxid und/oder Propylenoxid, mit primären und sekundären Aminen.

Das erfindungsgemäße Verfahren eignet sich vorteilhaft zur Herstellung quartärer Ammonium-Verbindungen aus Aminoalkoholen, Aminomercaptanen und deren Estern. Bevorzugt sind die Ester mit anorganischen Säuren, speziell Schwefelsäure und Phosphorsäure, sowie die Derivate dieser Ester. Die in Schritt a) eingesetzte Amin-Verbindung weist dann zusätzlich zur Aminofunktion wenigstens einen Substituenten auf, der ausgewählt ist unter Hydroxylgruppen, Mercaptogruppen, Estergruppen, Thioestergruppen und Kombinationen davon. So ermöglicht das erfindungsgemäße Verfahren erstmals die Herstellung von quartären Ammonium-Verbindungen aus Aminoalkoholen, Aminomercaptanen und deren Estern, die frei von Cl⁻, Br⁻, I⁻ und gleichzeitig frei von Monoalkylsulfatanionen und Monoalkylphosphatanionen sind.

Geeignete Aminoalkohole sind z. B. 2-Aminoethanol (= Monoethanolamin), 3-Amino-1-propanol, 2-Amino-1-propanol, 1-Amino-2-propanol, 2-Amino-3-phenylpropanol, 2-Amino-2-methyl-1-propanol, 2-Amino-1-butanol, 4-Amino-1-butanol, 2-Aminoisobutanol, 2-Amino-3-methyl-1-butanol, 2-Amino-3,3-dimethylbutanol, 1-Amino-1-pentanol, 5-Amino-1-pentanol, 2-Amino-1-pentanol, 2-Amino-4-methyl-1-pentanol, 2-Amino-3-methyl-1-pentanol, 2-Aminocyclohexanol, 4-Aminocyclohexanol, 3-(Aminomethyl)-3,5,5-trimethylcyclohexanol, 2-Amino-1,2-diphenylethanol, 2-Amino-1,1-diphenylethanol, 2-Amino-2-phenylethanol, 2-Amino-1-phenylethanol, 2-(4-Aminophenyl)-ethanol, 2-(2-Aminophenyl)-ethanol, 1-(3-Aminophenyl)ethanol, 2-Amino-1-hexanol, 6-Amino-1-hexanol, 6-Amino-2-methyl-2-heptanol, N-Methylisopropanolamin, N-Ethylisopropanolamin, N-Methylethanolamin, N-Ethylethanolamin, N-(3-Hydroxypropyl)-methylamin, N-(2-Hydroxyethyl)-dimethylamin, N-(3-Hydroxypropyl)-dimethylamin, N-(2-Hydroxyethyl)-diethylamin, N-(3-Hydroxypropyl)-diethylamin, 1-Ethylaminobutan-2-ol, 4-Methyl-4-aminopentan-2-ol, 2-(2-Aminoethoxy)-ethanol, N-(2-Hydroxyethyl)-piperazin, 1-Amino-2-indanol, N-(2-Hydroxyethyl)-anilin, Aminozucker, wie D-Glucoseamin, D-Galactoseamin, 4-Amino-4,6-didesoxy-α-D-glucopyranose, N-(2-Hydroxyethyl)-ethylendiamin, Diethanolamin, Dipropanolamin, Diisopropanolamin, 2-Amino-1,3-propandiol, 3-Amino-1,2-propandiol, und Mischungen davon.

Eine bevorzugte Ausführung des erfindungsgemäßen Verfahrens dient der Herstellung von quartären Ammonium-Verbindungen aus N-(Hydroxyalkyl)-dialkylaminen und speziell aus N-(2-Hydroxyethyl)-dimethylamin (Dimethylaminoethanol). Quartäre Ammonium-Verbindungen des N-(2-Hydroxyethyl)-dimethylamins, d. h. Cholin (= (2-Hydroxyethyl)-trimethylammoniumhydroxid) und andere Salze (Cholinium-Salze) haben in der Pharmazie, Futtermittelindustrie, als Nahrungsergänzung etc. weite Verbreitung gefunden, und es besteht ein entsprechend hoher Bedarf an halogenfreien Cholinium-Salzen.

Geeignete Amin-Verbindungen a), die wenigstens eine Mercaptogruppe aufweisen, sind z. B. 2-Mercaptoethylamin, 3-Mercaptopropylamin, 4-Mercaptobutylamin, N-(2-Mercaptoethyl)-methylamin, N-(2-Mercaptoethyl)-dimethylamin, etc.

Geeignete Amin-Verbindungen a) sind weiterhin die Ester von Aminoalkoholen und Aminothiolen mit anorganischen Säuren sowie deren Derivate. Dabei handelt es sich insbesondere um Ester des Dimethylaminoethanols mit Schwefelsäure, Phosphorsäure und deren Derivate. Das erfindungsgemäße Verfahren eignet sich somit speziell zur Herstellung von Cholin-O-phosphat (Phosphorylcholin), Cholin-O-sulfat (Sulfurylcholin) und O-Phosphatidylcholin (Lecithin). Die zuvor genannten Verbindungen weisen sämtlich Betainstruktur auf. Phosphorylcholin verbessert die Biokompatibilität von Materialien und findet daher Verwendung zur Beschichtung von Kontaktlinsen und in der modernen Medizin, z. B. für Implantate, Bypässe (Koronarstents), Kanülen, etc. Des Weiteren werden die genannten Verbindungen und ihre Derivate in der Nahrungsmittelindustrie, z. B. zur Herstellung von Babynahrung und Nahrungsergänzungen, sowie in der Kosmetik eingesetzt. Es besteht somit ein großer Bedarf an Verfahren zu ihrer möglichst reinen, speziell Halogenid-freien Herstellung.

Die zuvor genannten Amin-Verbindungen werden vorzugsweise einzeln eingesetzt. Sie können jedoch auch in Form von beliebigen Mischungen eingesetzt werden.

Zur erfindungsgemäßen Herstellung von ionischen Verbindungen, die wenigstens ein Kation mit einem quaternären sp³-hybridisierten Stickstoffatom umfassen, wird in einem ersten Reaktionsschritt a) eine Verbindung, die ein sp³-hybridisiertes Stickstoffatom enthält, mit einem Dialkylsulfat oder Trialkylphosphat unter Erhalt einer quartären Ammoniumverbindung umsetzt, die zumindest teilweise mehrwertige Anionen aufweist und anschließend in einem Schritt b) die in Schritt a) erhaltene ionische Verbindung einem Anionenaustausch unterzogen.

Erfindungsgemäß erfolgt die Umsetzung in Schritt a) bei einer erhöhten Temperatur, d. h. bei einer Temperatur, die oberhalb der Umgebungstemperatur liegt. Vorzugsweise beträgt die Temperatur in Schritt a) wenigstens 40 °C, besonders bevorzugt wenigstens 80 °C. Vorzugsweise erfolgt die Umsetzung in Schritt a) bei. einer Temperatur im Bereich von oberhalb 100 bis 220 °C, besonders bevorzugt von 120 bis 200 °C.

In einer bevorzugten Ausführung wird in Schritt a) zunächst die Amin-Verbindung mit dem Dialkylsulfat oder dem Trialkylphosphat bei einer Temperatur von höchstens 30 °C in Kontakt gebracht und anschließend das resultierende Gemisch zur weiteren Umsetzung auf eine Temperatur von wenigstens 40 °C, wie zuvor beschrieben, erwärmt. Bevorzugt erfolgt das in Kontakt bringen der Amin-Verbindung mit dem Dialkylsulfat oder Trialkylphosphat bei einer Temperatur von höchstens 20 °C, insbesondere bei einer Temperatur von höchstens 10 °C. Bevorzugt erfolgt das Inkontaktbringen der Amin-Verbindung mit dem Dialkylsulfat oder Trialkylphosphat portionsweise. Dazu kann das Amin oder das Dialkylsulfat/Trialkylphosphat vorgelegt und die jeweils andere Komponente portionsweise zugegeben werden. Bevorzugt werden beide Komponenten in flüssiger Form, z. B. in Form einer wässrigen Lösung eingesetzt. Unter einer wässrigen Lösung werden dabei Wasser und Gemische von Wasser mit wassermischbaren Lösungsmitteln verstanden.

Die Umsetzung in Schritt a) erfolgt in der Regel unter erhöhtem Druck. Bevorzugt erfolgt die Reaktion unter dem Eigendruck der Reaktionsmischung bei den Reaktionsbedingungen. Beim Einsatz flüchtiger Amine beträgt der Druck bei der Umsetzung in Schritt a) im Allgemeinen mindestens 1,5 bar, insbesondere mindestens 2 bar. Gewünschtenfalls kann der Druck bei der Umsetzung in Schritt a) bis 300 bar betragen. Geeignete druckfeste Reaktoren sind dem Fachmann bekannt und werden z. B. in Ullmanns Enzyklopädie der technischen Chemie, Band 1, 3. Auflage, 1951, S. 769 ff beschrieben. Im Allgemeinen wird für das erfindungsgemäße Verfahren ein Autoklav verwendet, der gewünschtenfalls mit einer Rührvorrichtung und/oder einer Innenauskleidung versehen sein kann.

Bei Einsatz wenigstens eines Dialkylsulfats als Alkylierungsmittel beträgt das Molmengenverhältnis von der zu alkylierenden Amin-Verbindung zu dem Dialkylsulfat vorzugsweise wenigstens 2:1. Besonders bevorzugt liegt das Molmengenverhältnis von der Amin-Verbindung zu dem Dialkylsulfat in einem Bereich von 1,8:1 bis 10:1, insbesondere 2,05:1 bis 5:1, speziell 2,1:1 bis 3:1.

Bei Einsatz wenigstens eines Trialkylphosphats als Alkylierungsmittel beträgt das Molmengenverhältnis von der zu alkylierenden Amin-Verbindung zu dem Trialkylphosphat beträgt vorzugsweise wenigstens 2:1. Besonders bevorzugt liegt das Molmengenverhältnis von der Amin-Verbindung zu dem Trialkylphosphat in einem Bereich von 3:1 bis 5:1.

Die Umsetzung der zu alkylierenden Verbindung mit dem Dialkylsulfat oder Trialkylphosphat kann in Substanz oder vorzugsweise in Gegenwart eines unter den Reaktionsbedingungen inerten Lösungsmittels erfolgen. Geeignete Lösungsmittel sind z. B. Wasser, wassermischbare Lösungsmittel, beispielsweise Alkohole, wie Methanol und Ethanol, und Mischungen davon. Bevorzugt wird als Lösungsmittel Wasser oder ein Lösungsmittelgemisch eingesetzt, das mindestens 30 Vol-%, bevorzugt mindestens 50 Vol-%, insbesondere mindestens 80 Vol-% Wasser umfasst.

Bei den in Schritt a) eingesetzten Dialkylsulfaten handelt es sich vorzugsweise um Di-C₁-C₁₀-alkylsulfate und insbesondere um Di-C₁-C₆-alkylsulfate, wie Dimethyl-, Diethyl-, Di-n-propyl-, Diisopropyl-, Di-n-butyl-, Diisobutyl-, Di-tert.-butyl-, Di-n-pentyl-, Diisopentyl-, Dineopentyl- und Di-n-hexylsulfat. Besonders bevorzugt werden Dimethylsulfat und Diethylsulfat eingesetzt.

Bei den in Schritt a) eingesetzten Trialkylphosphaten handelt es sich vorzugsweise um Tri-C₁-C₁₀-alkylphosphate und insbesondere um Tri-C₁-C₆-alkylphosphate, wie Trimethyl-, Triethyl-, Tri-n-propyl-, Triisopropyl-, Tri-n-butyl-, Triisobutyl-, Tri-tert.-butyl-, Tri-n-pentyl-, Triisopentyl-, Trineopentyl- und Tri-n-hexylphosphat. Besonders bevorzugt werden Trimethylphosphat und Triethylphosphat eingesetzt.

Gewünschtenfalls kann die Umsetzung in Schritt a) in Gegenwart wenigstens eines Inertgases erfolgen. Geeignete Inertgase sind beispielsweise Stickstoff, Helium und Argon.

Die Umsetzung in Schritt a) kann kontinuierlich oder diskontinuierlich erfolgen.

Aus dem in Schritt a) erhaltenen Reaktionsgemisch können die quartären Ammoniumsalze nach üblichen, dem Fachmann bekannten Verfahren isoliert werden. Dies gilt speziell, wenn die Umsetzung in Schritt b) in einem anderen Lösungsmittel erfolgen soll als die Alkylierung in Schritt a). Wurde zur Umsetzung in Schritt a) ein Lösungsmittel eingesetzt, so kann dieses durch Verdampfen, vorzugsweise unter verringertem Druck, entfernt werden. Da die erhaltenen ionischen Verbindungen nicht flüchtig sind, ist der eingesetzte Druckbereich in der Regel nicht kritisch. Sofern eine möglichst vollständige Entfernung des Lösungsmittels gewünscht ist, kann beispielsweise ein Feinvakuum von 10¹ bis 10⁻¹ Pa oder ein Hochvakuum von 10⁻¹ bis 10⁻⁵ Pa eingesetzt werden. Zur Druckerzeugung können übliche Vakuumpumpen, wie Flüssigkeitsstrahlvakuumpumpen, Dreh- und Sperrschiebervakuumpumpen, Membranvakuumpumpen, Diffusionspumpen etc. eingesetzt werden. Das Entfernen des Lösungsmittels kann zudem bei einer erhöhten Temperatur von bis zu 150 °C, bevorzugt bis zu 100 °C erfolgen.

Bevorzugt wird das in Schritt a) erhaltene Reaktionsgemisch ohne vorherige Isolierung zur Umsetzung in Schritt b) eingesetzt.

Der Anionenaustausch in Schritt b) kann durch Umprotonierung, Umsetzung mit einem Metallsalz, Ionenaustauschchromatographie, elektrolytisch oder eine Kombination dieser Maßnahmen erfolgen.

In einer ersten Ausführungsform wird die in Schritt a) des erfindungsgemäßen Verfahrens erhaltene quartäre Ammoniumverbindung, die zumindest teilweise mehrwertige Anionen aufweist, mit einer Säure, vorzugsweise Schwefelsäure oder Phosphorsäure, unter Protonenübertragung umgesetzt.

Bevorzugt wird zur Umprotonierung eine quartäre Ammoniumverbindung mit Sulfatanionen mit Schwefelsäure umgesetzt, wobei die entsprechenden Hydrogensulfate (Xⁿ⁻ =HSO₄⁻) erhalten werden. Die Umprotonierung erfolgt vorzugsweise mit 100 %iger H₂SO₄. Das Molmengenverhältnis von H₂SO₄ zu SO₄²⁻ beträgt vorzugsweise ≥ 1:1 und liegt beispielsweise in einem Bereich von 1:1 bis 2:1.

Des Weiteren bevorzugt wird zur Umprotonierung eine quartäre Ammoniumverbindung mit Phosphat- oder Monoalkylphosphatanionen mit Phosphorsäure umgesetzt, wobei die entsprechenden Hydrogenphosphate (Xⁿ⁻ = HPO₄²⁻) und/oder Dihydrogenphosphate (Xⁿ⁻ = H₂PO₄⁻) erhalten werden. Das Molmengenverhältnis von H₃PO₄ zu auszutauschenden Anionen beträgt vorzugsweise ≥ 1:1 und liegt beispielsweise in einem Bereich von 1:1 bis 2:1.

In einer weiteren Ausführungsform erfolgt der Anionenaustausch in Schritt b) durch die Umsetzung mit einem Metallsalz. Vorzugsweise erfolgt diese Umsetzung in einem Lösungsmittel, aus dem ein aus dem Metall des Metallsalzes und dem Sulfatanion gebildetes Metallsulfat auskristallisiert. Für diese Variante des Anionenaustauschs können auch die zuvor beschriebenen Hydrogensulfate eingesetzt werden. Bei dem Kation des Metallsalzes handelt es sich vorzugsweise um Alkalimetall-, Erdalkalimetall-, Blei- oder Silberionen. Das Anion des Metallsalzes ist ausgewählt unter den zuvor genannten Anionen Xⁿ⁻, wobei es sich insbesondere um ein von Cl⁻, Br⁻, I⁻ Monoalkylsulfat und Monoalkylphosphat verschiedenes Anion handelt. In einer geeigneten Vorgehensweise wird eine Lösung des Metallsalzes mit einer Lösung der quartären Amin-Verbindung in Kontakt gebracht. Geeignete Lösungsmittel sind z. B. Wasser, wassermischbare Lösungsmittel, beispielsweise Alkohole, wie Methanol und Ethanol, und Mischungen davon. Die Reaktionstemperatur liegt vorzugsweise in einem Bereich von -10 bis 100 °C, insbesondere 0 bis 80 °C.

In einer weiteren Ausführungsform erfolgt der Anionenaustausch in Schritt b) durch Ionenaustauschchromatographie. Dazu eignen sich prinzipiell die dem Fachmann bekannten basischen Ionenaustauscher, die wenigstens eine an eine Festphase immobilisierte Base aufweisen. Die Festphase dieser basischen Ionenaustauscher umfasst beispielweise eine Polymermatrix. Dazu zählen z. B. Polystyrolmatrices, die neben Styrol wenigstens ein vernetzendes Monomer, z. B. Divinylbenzol, sowie gegebenenfalls weitere Comonomere einpolymerisiert enthalten. Geeignet sind weiterhin Polyacrylmatrices, die durch Polymerisation wenigstens eines (Meth)acrylats, wenigstens eines vernetzenden Monomers sowie gegebenenfalls weiterer Comonomere erhalten werden. Geeignete Polymermatrices sind auch Phenol-Formaldehyd-Harze und Polyalkylamin-Harze, die beispielsweise durch Kondensation von Polyaminen mit Epichlorhydrin erhalten werden.

Die an die Festphase direkt oder über eine Spacergruppe gebundenen so genannten Ankergruppen (deren locker gebundene Gegenionen gegen gleichsinnig geladene Ionen ausgetauscht werden können) sind vorzugsweise ausgewählt unter stickstoffhaltigen Gruppen, vorzugsweise tertiären und quartären Aminogruppen.

Geeignete funktionelle Gruppen sind z. B. (geordnet nach abnehmender Basizität):

| | |
|---|---|
| -CH₂N⁺(CH₃)₃ OH⁻ | z. B. Duolite A 101 |
| -CH₂N⁺(CH₃)₂CH₂CH₂OH OH⁻ | z. B. Duolite A 102 |
| -CH₂N(CH₃)₂ | z. B. Amberlite IRA 67 |
| -CH₂NHCH₃ | |
| -CH₂NH₂ | z. B. Duolite A 365 |

Für das erfindungsgemäße Verfahren eignen sich sowohl stark als auch schwach basische Ionenaustauscher, bevorzugt sind stark basische Ionenaustauscher in OH-Form. Unter den schwach basischen Ionenaustauschern sind solche, die tertiäre Aminogruppen aufweisen bevorzugt. Stark basische Ionenaustauscher weisen in der Regel quaternäre Ammoniumgruppen als Ankergruppen auf. Für das erfindungsgemäße Verfahren geeignete kommerziell erhältliche Ionenaustauscher sind z. B. Amberlyst® A21 (Dimethylamino-funktionalisiert, schwach basisch), Amberlyst® A27 (quaternäre Ammoniumgruppen, stark basisch) und, Ambersep® 900 OH (stark basisch). Zum Ionenaustausch werden die Ionenaustauscher zunächst mit den gewünschten Anionen Xⁿ⁻ beladen und anschließend mit den ionische Verbindungen auf Basis von Sulfatanionen oder Phosphatanionen (bzw. Hydrogensulfatanionen, Hydrogenphosphatanionen und/oder Dihydrogenphosphatanionen) in Kontakt gebracht.

In einer weiteren Ausführungsform erfolgt der Anionenaustausch in Schritt b) durch Elektrolyse (Elektrodialyse). Durch den Einsatz von Elektrolysezellen mit lonenaustauschmembranen gelingt somit beispielsweise die Herstellung von Basen aus den entsprechenden Salzen. Geeignete Elektrodialysezellen und Membranen für den Anionenaustausch sowie bipolare Membranen für den gleichzeitigen Austausch von Kationen und Anionen sind bekannt und kommerziell erhältlich (z. B. von FuMA-Tech St. Ingbert, Deutschland; Asahl Glass; PCA -Polymerchemie Altmeier GmbH und PCCell GmbH, Lebacher Straße 60, D-66265, Heusweiler, Germany). Auch bei dieser Ausführungsform erweist es sich als besonders vorteilhaft für die Lebensdauer der eingesetzten Elektrolysevorrichtungen, speziell der Membranen, dass in Schritt a) des erfindungsgemäßen Verfahrens quartäre Ammonium-Verbindungen mit mehrwertigen, nicht korrosiven Anionen gebildet werden.

Geeignete Elektrolysezellen für den Anionenaustausch sind zum einen Zellen, bei denen die Elektrodenkompartimente durch eine Membran voneinander getrennt sind. Geeignete Membranen sind beispielsweise Membranen auf Basis von Perfluoropolymeren. Geeignete Elektrolysezellen für den Anionenaustausch sind auch solche, bei denen die Elektrodenkompartimente nicht durch eine Membran voneinander getrennt sind. Dazu zählen beispielsweise "capillary gap cells" (CGC), die beispielsweise aus einem bipolaren Stapel Elektrodenscheiben bestehen, die beispielweise aus Graphit oder Graphit-modifizierten Kunststoffen bestehen. Geeignet sind auch "solid polymer electrolyte (SPE) cells", die kein zusätzliches Elektrolyt benötigen.

In einer Ausgestaltung des Verfahrens zur Herstellung von quartären Ammoniumhydroxiden kann man beispielsweise eine nach Schritt a) des erfindungsgemäßen Verfahrens erhaltene quartäre Ammonium-Verbindung mit Sulfat-, Monolkylphosphat- oder Phosphatanionen elektrolytisch in das entsprechende quartäre Ammoniumhydroxid überführen. Gewünschtenfalls kann sich an den elektrolytischen Anionenaustausch eine Ionenaustauschchromatographie anschließen. Somit lassen sich hochreine quartäre Ammoniumverbindungen erzielen, die unerwünschte Anionen nur noch in äußerst geringen Konzentrationen oder unterhalb der nachweisbaren Menge erhalten.

Das erfindungsgemäße Verfahren ermöglicht erstmals die Herstellung von Verbindungen der allgemeinen Formel b B^{m+} x Xⁿ (I), wie zuvor definiert, die frei von Cl⁻, Br⁻, I⁻ und gleichzeitig frei von Monoalkylsulfatanionen und Monoalkylphosphatanionen sind. Vorzugsweise erfolgt zur Herstellung von Verbindungen der Formel I mit äußerst geringem Restgehalt an Halogenidionen die Umsetzung in den Schritten a) und b) unter Ausschluss von Halogenidionen und von Materialien, die diese freisetzen. So können zur Umsetzung Reagenzien, Lösungsmittel, Inertgase etc. eingesetzt werden, die im Wesentlichen frei von Halogenidionen sind. Derartige Komponenten sind kommerziell erhältlich oder können durch übliche, dem Fachmann bekannte Reinigungsverfahren hergestellt werden. Dazu zählen z. B. Adsorptions-, Filtrations- und Ionenaustauschverfahren. Gewünschtenfalls können auch die in den Schritten a) und b) eingesetzten Vorrichtungen vor ihrem Einsatz von Halogenidionen befreit werden, z. B. durch Spülen mit halogenidfreien Lösungsmitteln. Nach dem erfindungsgemäßen Verfahren können Verbindungen der allgemeinen Formel I erhalten werden, worin Xⁿ⁻ für OH⁻ steht und die einen Gesamtgehalt an Halogenidionen von höchstens 100 ppm, bevorzugt von höchstens 10 ppm und insbesondere von höchstens 1 ppm aufweisen. Des Weiteren können solche Verbindungen erhalten werden, die einen Gesamtgehalt an Monoalkylsulfatanionen von höchstens 100 ppm, bevorzugt von höchstens 10 ppm und insbesondere von höchstens 1 ppm aufweisen.

Ein weiterer Gegenstand der Erfindung ist ein Verfahren zur Herstellung von hochreinen wässrigen Tetraalkylammoniumhydroxid-Lösungen, wobei man, jeweils in Wasser und unter Ausschluss Verbindungen und von Materialien, die Halogenidionen enthalten oder diese freisetzen,
a) eine Trialkylamin-Verbindung mit einem Dialkylsulfat unter Einsatz beider Alkylgruppen des Dialkylsulfats umsetzt, wobei eine hochreine wässrige Tetraalkylammoniumsalz-Lösungen erhalten wird, und
b) die in Schritt a) erhaltene Tetraalkylammoniumsalz-Lösung einem elektrolytischen Anionenaustausch oder einer Ionenaustauschchromatographie zum Ersatz der Sulfatanionen durch Hydroxidionen unterzieht.

Bevorzugt wird in dem zuvor genannten Verfahren Trimethylamin mit Dimethylsulfat unter Erhalt einer hochreinen wässrigen Tetramethylammoniumhydroxid-Lösung umgesetzt.

Hochreine Tetramethylammoniumhydroxid-Lösungen, mit einem Gesamtgehalt an Halogenidionen von höchstens 100 ppb in der Elektronik-Industrie, eignen sich für die Verwendung zur Behandlung von Substraten elektronischer Bauteile, insbesondere zur Behandlung von Halbleiter-Bauelementen. So eignen sich Tetramethylammoniumhydroxid-Lösungen vorteilhaft zum Ätzen von Siliziumwafern. Sie sind befähigt zu einem sogenannten anisotropen Ätzen, bei dem die Ätzrate abhängig von der Kristallrichtung des Siliziums ist. Die einzelnen Kristallebenen können dabei als lateraler Ätzstopper beim Abtragen des Siliziums wirken, wodurch eine hohe Strukturgenauigkeit erreicht wird.

Die Erfindung wird anhand der folgenden nicht einschränkenden Beispiele näher erläutert.

### Beispiele

### Beispiel 1:

### a) Herstellung von N,N-Dimethylpiperidiniumsulfat durch Umsetzung in Wasser

In einem 250 ml Kolben mit Tropftrichter, Rückflusskühler und Magnetrührer wurden 172,9 ml destilliertes Wasser und 31,7 g, (0,252 mol) N-Methylpiperidin vorgelegt und unter Rühren 15,1 g (0,12 mol) Dimethylsulfat zugegeben, wobei die Innentemperatur durch Eiskühlung auf unter 30 °C gehalten wurde. Die Reaktionsmischung wurde anschließend 6 h unter Eigendruck im Autoklaven auf 120 °C erhitzt. Die so erhaltene Lösung wurde nach dem Abkühlen direkt in Schritt b) bzw. Schritt c) eingesetzt.

### b) Herstellung von N,N-Dimethylpiperidiniumhydroxid durch Umsetzung mit Bariumhydroxid

In einem 500 ml Rührkolben, der mit einem Tropftrichter ausgestattet war, wurden 32,07 g (0,1017 mol) Bariumhydroxid (Octahydrat) und 258,3 g Wasser vorgelegt und auf 40 °C erwärmt. Über den Tropftrichter wurde innerhalb von 30 min. ein Teil der in Schritt a) hergestellten wässrigen Lösung des Sulfats 38,5 g (0,1017 mol) zugetropft. Sofort nach Beginn der Zugabe bildete sich ein schneeweißer feinpulvriger Niederschlag von Bariumsulfat. Nach Beendigung der Zugabe wurde das Reaktionsgemisch 7 h bei 40 °C nachgerührt, abgekühlt und der Niederschlag wurde über einen Blaubandfilter abgesaugt. Man erhielt 274 g einer wasserklaren Lösung von N,N-Dimethylpiperidiniumhydroxid. Titration der Lösung mit 0,1 HCl ergab eine Hydroxidzahl von 8,45 % was einer Ausbeute von 89 % der Theorie entspricht. Die Sulfatkonzentration war < 1 ppm.

### c) Herstellung von N,N-Dimethylpiperidiniumhydroxid durch Umsetzung mit einem OH-beladenen Ionentauscher

70 g einer 10 gew.-%igen wässrigen Lösung von N,N-Dimethylpiperidiniumsulfat, erhältlich durch Verdünnen der in Schritt a) hergestellten wässrigen Lösung mit entionisiertem Wasser, wurde mit 60 ml eines stark basischen Ionentauschers (Ambersep® 900 OH) in der OH-Form (Beladung 0,59 val/l) versetzt und die Mischung wurde 24 h bei Raumtemperatur geschüttelt. Anschließend wurde der Ionentauscher abfiltriert. Man erhielt auf diese Weise eine etwa 10 gew.-%ige wässrige Lösung von N,N-Dimethylpiperidiniumhydroxid. Eine Analyse der Lösung auf Sulfat ergab einen Sulfatgehalt < 1 ppm, bezogen auf Lösung.

### Beispiel 2:

### a) Herstellung von Tetramethylammoniumsulfat

In einem 250 ml Kolben mit Tropftrichter und Magnetrührer wurden 100 ml destilliertes Wasser und 12,6 g (0,1 mol) Dimethylsulfat vorgelegt und unter Rühren innerhalb von 30 Minuten 50,0 g (0,42 mol) Trimethylamin (als 50 %ige Lösung in Wasser) zugetropft, wobei die Innentemperatur durch Eiskühlung auf 20 °C gehalten wurde. Die Reaktionsmischung wurde anschließend in einen 300 ml Rührautoklaven überführt und dieser 6 h auf 120 °C erhitzt. Dabei stieg der Innendruck auf 9,1 bar an. Nach dem Abkühlen und Entspannen wurde der Rohaustrag am Rotationsverdampfer eingedampft und der erhaltene Rückstand am Ölpumpenvakuum bei 50 °C getrocknet und anschließend mit 300 ml Aceton bei Raumtemperatur 2 h gerührt. Danach wurde das Aceton abgesaugt, der resultierende Feststoff nochmals mit 100 ml Aceton gewaschen und anschließend am Ölpumpenvakuum getrocknet. Es wurden 26,56 g Tetramethylammoniumsulfat mit einem Wassergehalt von 10,5 Gew.-% erhalten. Dies entspricht einer Ausbeute von 97 % der Theorie.

### b) Herstellung von Tetramethylammoniumhydroxid mittels Ionentauscher

Eine 20 gew.-%ige Lösung von Tetramethylammoniumsulfat in Wasser (entsprechend 79000 mg Sulfat/kg) wird kontinuierlich (680 ml/h) über eine mit dem Anionenaustauscher Ambersep® 900 OH gefüllte Säule (Länge 1 m, Volumen 646 ml) gegeben. Der maximal gemessene Sulfatgehalt des Eluats betrug < 1 mg/kg, der maximal gemessene Chloridgehalt 7 mg/kg. Der Anionenaustauscher ließ sich mit hochreiner NaOH regenerieren und erneut zum Anionenaustausch einsetzen.

### c) Herstellung von Tetramethylammoniumhydroxid mittels Elektrolyse

1380 g einer 20 gew.-%igen Lösung von Tetramethylammoniumsulfat, erhältlich nach der Vorschrift in Schritt a), gab man in die Mittelkammer einer Elektrolyseanordnung mit drei Kammern. Die Mittelkammer war anodenseitig durch eine FT-FAP-Anionentauschermembran (Firma FuMA-Tech) vom Anolyten getrennt. Kathodenseitig war die Mittelkammer mit einer CMV-Kationentauschermembran (Asahi-Glass) vom Katholythen (Produkt) getrennt. Als Anode diente eine DSA, als Kathode diente Edelstahl. Im Anolyten legte man 5000 g einer 0,02 molaren wässrigen Schwefelsäure vor. Im Kathodenkreislauf legte man 1250 g einer 1 gew.-%igen Lösung von Tetramethylammoniumhydroxid (electronic grade) in Reinstwasser vor. Man elektrolysierte 6 h bei 81,2 Ah. Dann entnahm man dem Katholythen 1592 g einer 11,7 gew.-%igen Lösung von Tetramethylammoniumhydroxid. Die Stromausbeute bezogen auf den Katholythen betrug 61,2 %. Der Sulfatgehalt im Katholythen betrug 3 ppm, der Eisenwert etwa 10 ppp. Die Konzentration von Tetramethylammoniumsulfat im Eduktkreislauf lag bei 0,1 Gew.-%. Die Endkonzentration an Schwefelsäure im Anolyten betrug 2,2 Gew.-%.

### Beispiel 3:

### a) Herstellung von Tetramethylammoniumphoshpat

In einem 250 ml Kolben mit Tropftrichter und Magnetrührer wurden 100 ml destilliertes Wasser und 14,0 g (0,1 mol) Trimethylphosphat vorgelegt und unter Rühren 60,0 g (0,508 mol) Trimethylamin zugetropft, wobei die Innentemperatur durch Eiskühlung auf 20 °C gehalten wurde. Die Reaktionsmischung wurde anschließend in einen 300 ml Rührautoklaven überführt und dieser unter Rühren 6 h auf 180 °C erhitzt. Dabei stieg der Innendruck auf 25 bar an. Nach dem Abkühlen und Entspannen wurde der Rohaustrag am Rotationsverdampfer eingedampft und der erhaltene Rückstand am Ölpumpenvakuum bei 50 °C getrocknet, wobei eine teilkristalline Substanz erhalten wurde. Diese wurde mit 500 ml Aceton bei Raumtemperatur 24 h gerührt. Anschließend wurde unter Stickstoffatmosphäre das Aceton abgesaugt, der resultierende Feststoff nochmals mit 100 ml Aceton gewaschen und anschließend am Ölpumpenvakuum getrocknet. Es wurden 29,51 g (92,3 % der Theorie) Produkt erhalten. Der in Schritt a) erhaltene Feststoff wurde in entionisiertem Wasser mit einer Konzentration von 20 Gew.-% gelöst. Die Lösung wurde über eine Säule mit einem basischen Anionenaustauscher geleitet. Das Eluat enthielt Tetramethylammoniumhydroxid. Der Phosphatgehalt der Lösung, bestimmt durch Elementaranalyse, lag unterhalb 1 ppm.

### Beispiel 4:

### Herstellung von N,N-Dimethylmorpholiniumhydroxid

In einem 250 ml Kolben mit Tropftrichter und Magnetrührer wurden 100 ml destilliertes Wasser und 14,0 g (0,1 mol) Trimethylphosphat vorgelegt und unter Rühren 60,0 g (0,508 mol) N-Methylmorpholin zugetropft, wobei die Innentemperatur durch Eiskühlung auf 20 °C gehalten wurde. Die Reaktionsmischung wurde anschließend in einen 300 ml Rührautoklaven überführt und dieser unter Rühren 6 h auf 180 °C erhitzt. Dabei stieg der Innendruck auf 25 bar an. Nach dem Abkühlen und Entspannen wurde der Rohaustrag am Rotationsverdampfer eingedampft und der erhaltene Rückstand am Ölpumpenvakuum bei 50 °C getrocknet, wobei eine teilkristalline Substanz erhalten wurde. Diese wurde mit 500 ml Aceton bei Raumtemperatur 24 h gerührt. Anschließend wurde unter Stickstoffatmosphäre das Aceton abgesaugt, der resultierende Feststoff nochmals mit 100 ml Aceton gewaschen und anschließend am Ölpumpenvakuum getrocknet. Es wurden 29,51 g N,N-Dimethylmorpholiniumsulfat (92,3 % der Theorie) erhalten. Der Feststoff wurde in Wasser gelöst und analog Beispiel 1b) mit Bariumhydroxid umgesetzt. Man erhielt auf diese Weise eine wässrige Lösung von N,N-Dimethylmorpholiniumhydroxid mit einem Sulfatgehalt (berechnet aus dem elementaranalytisch bestimmten Schwefelgehalt) < 270 ppm. Der Bariumgehalt der Lösung betrug ebenfalls < 1 ppm.

### Beispiel 5:

### a) Herstellung von C₁₄-Alkyltrimethylammoniumsulfat

In einem 250 ml Kolben mit Tropftrichter und Magnetrührer wurden 48,2 g (0,2 mol) C₁₄-Alkyldimethylamin in 100 ml destilliertem Wasser vorgelegt und 12,6 g (0,1 mol) Dimethylsulfat zugegeben, wobei die Innentemperatur durch Eiskühlung auf 20 °C gehalten wurde. Die Reaktionsmischung wurde anschließend in einen 300 ml Rührautoklaven überführt und dieser unter Rühren 6 h auf 180 °C erhitzt. Dabei stieg der Innendruck auf 7,6 bar an. Nach dem Abkühlen und Entspannen wurde der Rohaustrag am Rotationsverdampfer eingedampft und der erhaltene Rückstand am Ölpumpenvakuum bei 50 °C getrocknet. Es wurden 52,3 g (86 % der Theorie) Produkt erhalten.
¹H-NMR (ppm, D₂O): 3,3 (m, 2H), 3,1 (s, 9H), 1,7 (m, 2H), 1,3 - 1,5 (m, 22H), 0,8 (t 3H)

### b) Herstellung von C₁₄-Alkyltrimethylammoniumhydroxid

Der Feststoff kann analog Beispiel 1 b) durch Umsetzung mit Bariumhydroxid in wässriger Lösung oder analog Beispiel 1 c) mittels Ionentauscher in das C₁₄-Alkyltrimethylammoniumhydroxid überführt werden.

### Beispiel 6:

### Herstellung von Bis-(trimethylethanolammonium)sulfat (Bis-Cholinsulfat)

19,6 g (0,22 mol) Dimethylaminoethanol wurden in 100 ml Methanol bei Raumtemperatur vorgelegt und unter Rühren 12,6 g (0,1 mol) Dimethylsulfat zugegeben, wobei die Innentemperatur durch Eiskühlung auf unter 30 °C gehalten wurde. Man ließ die Reaktionsmischung 30 min. bei 30 °C nachrühren und überführte sie anschließend in einen 300 ml-Rührautoklaven, der unter Rühren 10 h auf 130 °C erhitzt wurde. Dabei stieg der Innendruck auf 16 bar an. Nach dem Abkühlen und Entspannen wurde der Rohaustrag am Rotationsverdampfer eingedampft, wobei ein weißer Feststoff erhalten wurde. Dieser wurde mit Aceton gewaschen und anschließend am Ölpumpenvakuum getrocknet. Es wurden 27,93 g Bis-(trimethylethanolammonium)sulfat (93 % der Theorie) erhalten.

### Beispiel 7:

### a) Herstellung von Bis-(trimethylethanolammonium)sulfat durch Umsetzung in Wasser

In einem Reaktionskolben, der mit Tropftrichter, Rückflusskühler und Magnetrührer ausgestattet war, wurden bei Raumtemperatur 280,35 g (3,15 mol) Dimethylaminoethanol in 1500 ml Wasser vorgelegt und unter Rühren 189,1 g (1,5 mol) Dimethylsulfat im Verlauf von 30 min. zugegeben, wobei die Innentemperatur durch Eiskühlung auf unter 30 °C gehalten wurde. Die Reaktionsmischung wurde anschließend 10 h unter Eigendruck im Autoklaven auf 130 ° C erhitzt. Der Reaktionsaustrag wurde mittels eines Dünnschichtverdampfers vom Wasser befreit (T = 140 °C, 540 ml/h, Übergang 99°). Überschüssiges Dimethylaminoethanol wurde im Vakuum (2 mbar, 80 °C) entfernt. Das erhaltene Bis-(trimethylethanolammonium)sulfat wurde unmittelbar in Schritt b) zum Anionenaustausch eingesetzt.

### b) Herstellung von Bis-(trimethylethanolammonium)hydroxid durch Umsetzung mit Bariumhydroxid.

In einem Rührkolben, der mit einem Tropftrichter ausgestattet war, wurden 1190 g (1,485 mol) Bis-(trimethylammonium)sulfat als etwa 45 %ige Lösung in Wasser in 3200 g Wasser vorgelegt und 473 g (1,5 mol) Bariumhydroxid (Octahydrat) auf ein Mal zu der Lösung gegeben. Es bildete sich eine weiße Suspension, die 5 h bei 50 °C gerührt wurde. Nach Zugabe von 45 g Aktivkohle wurde die Suspension nochmals 2 h gerührt und der Niederschlag über einen Blaubandfilter abgesaugt. Man erhielt 4361 g einer Lösung von Bis-(trimethylethanolammonium)hydroxid (87,7 % Ausbeute). Eine Analyse der Lösung auf Sulfat ergab einen Sulfatgehalt von 36 ppm.

### c) Herstellung von Trimethylethanolammoniumsalicylat

In einem Rührkolben wurden 2000 g (1,195 mol) Trimethylethanolammoniumhydroxid-Lösung (7,23 % OH⁻) bei Raumtemperatur mit 164,9 g (1,195 mol) Salicylsäure versetzt. Die resultierende Lösung wurde auf 50 °C erwärmt und 2 h bei dieser Temperatur gerührt. Nach Einengen unter Vakuum (60 °C, 2 mbar) wurde das Trimethylethanolammoniumsalicylat als weißer Feststoff erhalten (282,4 g = 98 % Ausbeute).

### Elementaranalyse:

| Element | Ist-% | Soll-% |
|---|---|---|
| C | 59,3 | 59,75 |
| O | 27,1 | 26,55 |
| N | 6,0 | 5,8 |
| H | 7,9 | 7,9 |

### Beispiel 8:

### Herstellung von Bis-(dimethyldiethanolammonium)sulfat

Bei Raumtemperatur wurden 26,62 g (0,22 mol) N-Methyldiethanolamin in 100 ml Methanol vorgelegt und unter Rühren 12,6 g (0,1 mol) Dimethylsulfat zugegeben, wobei die Innentemperatur durch Eiskühlung auf unter 30 °C gehalten wurde. Die Reaktionsmischung wurde weitere 30 min. bei Raumtemperatur gerührt und anschließend in einen Autoklaven überführt. Nach 10 h Umsetzung im Autoklaven bei einer Temperatur von 160 °C bei einem Innendruck von 7 bar ließ man abkühlen, entspannte den Autoklaven und entfernte das Lösungsmittel im Vakuum (60 °C, 2 mbar). Es wurden 34,2 g Bis-(dimethyldiethanolammonium)sulfat (94 % der Theorie) erhalten.

## Patentansprüche

1. Verfahren zur Herstellung einer quartären Ammonium-Verbindung, bei dem man
a) eine Amin-Verbindung, die wenigstens ein sp³-hybridisiertes Stickstoffatom enthält, mit einem Dialkylsulfat oder Trialkylphosphat unter Erhalt einer quartären Ammoniumverbindung umsetzt, die zumindest teilweise mehrwertige Anionen aufweist, und
b) die in Schritt a) erhaltene quartäre Ammoniumverbindung einem Anionenaustausch unterzieht.

2. Verfahren nach Anspruch 1, bei dem man in Schritt a) die Amin-Verbindung mit einem Dialkylsulfat unter Einsatz beider Alkylgruppen des Dialkylsulfats umsetzt, wobei eine quartäre Ammoniumverbindung mit Sulfatanionen erhalten wird.

3. Verfahren nach Anspruch 1, bei dem man in Schritt a) die Amin-Verbindung mit einem Trialkylphosphat unter Einsatz von wenigstens zwei der Alkylgruppen des Trialkylphosphats umsetzt, wobei eine quartäre Ammoniumverbindung mit Phosphatanionen und/oder Monoalkylphoshatanionen erhalten wird.

4. Verfahren nach einem der vorhergehenden Ansprüche, wobei die in Schritt a) eingesetzte Amin-Verbindung ausgewählt ist unter Verbindungen der Formel NR¹R²R³, worin R¹, R² und R³ unabhängig voneinander ausgewählt sind unter Wasserstoff, Alkyl, Cycloalkyl, Heterocycloalkyl, Aryl und Hetaryl, wobei wenigstens zwei der Reste R¹, R² und R³ gemeinsam mit dem N-Atom, an das sie gebunden sind, auch Teil einer polycyclischen Verbindung sein können.

5. Verfahren nach einem der vorhergehenden Ansprüche, wobei die in Schritt a) eingesetzte Amin-Verbindung wenigstens einen weiteren Substituenten trägt, der ausgewählt ist unter Hydroxylgruppen, Mercaptogruppen, Estergruppen, Thioestergruppen und Kombinationen davon.

6. Verfahren nach Anspruch 5, wobei die in Schritt a) eingesetzte Amin-Verbindung ausgewählt ist unter Aminoalkoholen, Aminothiolen und deren Estern mit anorganischen Säuren, insbesondere Cholin-O-phosphat, Cholin-O-sulfat und O-Phosphatidylcholin.

7. Verfahren nach einem der vorhergehenden Ansprüche, wobei die erhaltene ionische Verbindung wenigstens ein Anion Xⁿ⁻ umfasst, worin n für eine ganze Zahl entsprechend der Wertigkeit des Anions steht und das ausgewählt ist unter OH⁻, HSO₄⁻, NO₂⁻, NO₃⁻, CN⁻, OCN⁻, NCO⁻, SCN⁻, NCS⁻, PO₄³⁻, HPO₄²⁻, (H₂PO₄⁻), H₂PO₃⁻, HPO₃²⁻, BO₃³⁻, (BO₂)₃³⁻, B₅O₆⁻, B₅O₈⁻, B₅H₄O₁₀⁻, [BF₄]⁻, [BCl₄]⁻, [B(C₆H₅)₄]⁻, [PF₆]⁻, [SbF₆]⁻, [AsF₆]⁻, [AlCl₄]⁻, [AlBr₄]⁻, [ZnCl₃]⁻, Dichlorocupraten(I) und (II), CO₃²⁻, HCO₃⁻, F⁻, (R'-COO)-, R'₃SiO⁻, (R'-SO₃)⁻ und [(R'-SO₂)₂N]⁻, worin R' für Alkyl, Cycloalkyl oder Aryl steht.

8. Verfahren nach einem der vorhergehenden Ansprüche, wobei die Umsetzung in Schritt a) bei einer Temperatur wenigstens 40 °C, bevorzugt wenigstens 80 °C, insbesondere im Bereich von 100 bis 220 °C erfolgt.

9. Verfahren nach einem der vorhergehenden Ansprüche, wobei man in Schritt a) zunächst die Amin-Verbindung mit dem Dialkylsulfat oder dem Trialkylphosphat bei einer Temperatur von höchstens 30 °C in Kontakt bringt und anschließend das resultierende Gemisch auf eine Temperatur von wenigstens 40 °C erwärmt.

10. Verfahren nach einem der vorhergehenden Ansprüche, wobei die Umsetzung in Schritt a) in einem organischen Lösungsmittel, in Wasser oder in einer Mischung davon durchgeführt wird.

11. Verfahren nach Anspruch 10, wobei das Lösungsmittel mindestens 30 Vol.-% Wasser umfasst.

12. Verfahren nach einem der vorhergehenden Ansprüche, wobei die Umsetzung in Schritt a) in Gegenwart eines Inertgases erfolgt.

13. Verfahren nach einem der vorhergehenden Ansprüche, wobei die Verfahrensschritte a) und b) in Abwesenheit von Halogenidionen durchgeführt werden.

14. Verfahren nach einem der vorhergehenden Ansprüche, wobei der Anionenaustausch in Schritt b) durch Umprotonierung, Umsetzung mit einem Metallsalz, Ionenaustauschchromatographie, elektrolytisch oder eine Kombination davon erfolgt.

15. Verfahren nach Anspruch 14, wobei die Umsetzung mit dem Metallsalz in einem Lösungsmittel durchgeführt wird, aus dem ein aus dem Metall des Metallsalzes und dem Sulfatanion gebildetes Metallsulfat auskristallisiert.

16. Verfahren, wie in einem der Ansprüche 1 bis 15 definiert, zur Herstellung von hochreinen wässrigen Tetraalkylammoniumhydroxid-Lösungen, wobei man, jeweils in Wasser und unter Ausschluss Verbindungen und von Materialien, die Halogenidionen enthalten oder diese freisetzen,
a) eine Trialkylamin-Verbindung mit einem Dialkylsulfat oder Trialkylphosphat umsetzt, wobei eine hochreine wässrige Tetraalkylammoniumsalz-Lösung erhalten wird, und
b) die in Schritt a) erhaltene Tetraalkylammoniumsalz-Lösung einem elektrolytischen Anionenaustausch oder einer Ionenaustauschchromatographie zum Ersatz der Anionen durch Hydroxidionen unterzieht.

## Claims

1. A process for preparing a quaternary ammonium compound, which comprises
a) reacting an amine compound comprising at least one sp³-hybridized nitrogen atom with a dialkyl sulfate or trialkyl phosphate to give a quaternary ammonium compound which has at least some polyvalent anions, and
b) subjecting the quaternary ammonium compound obtained in step a) to an anion exchange.

2. The process according to claim 1, wherein the amine compound is reacted in step a) with a dialkyl sulfate with participation of both alkyl groups of the dialkyl sulfate to give a quaternary ammonium compound having sulfate anions.

3. The process according to claim 1, wherein the amine compound is reacted in step a) with a trialkyl phosphate with participation of at least two of the alkyl groups of the trialkyl phosphate to give a quaternary ammonium compound having phosphate anions and/or monoalkylphosphate anions.

4. The process according to any of the preceding claims, wherein the amine compound used in step a) is selected from among compounds of the general formula NR¹R²R³, where R¹, R² and R³ are selected independently of one another from among hydrogen, alkyl, cycloalkyl, heterocycloalkyl, aryl and hetaryl, with at least two of the radicals R¹, R² and R³ together with the N atom to which they are bound also being able to be part of a polycyclic compound.

5. The process according to any of the preceding claims, wherein the amine compound used in step a) bears at least one further substituent selected from among hydroxyl groups, mercapto groups, ester groups, thioester groups and combinations thereof.

6. The process according to claim 5, wherein the amine compound used in step a) is selected from among amino alcohols, amino thiols and esters thereof with inorganic acids, in particular choline O-phosphate, choline 0-sulfate and 0-phosphatidylcholine.

7. The process according to any of the preceding claims, wherein the ionic compound obtained comprises at least one anion Xⁿ⁻ in which n is an integer corresponding to the valence of the anion and is selected from among OH⁻, HSO₄⁻, NO₂⁻, NO₃⁻, CN⁻, OCN⁻, NCO⁻, SCN⁻, NCS⁻, PO₄³⁻, HPO₄²⁻, (H₂PO₄⁻), H₂PO₃⁻, HPO₃²⁻, BO₃³⁻, (BO₂)₃³⁻, B₅O₆⁻, B₅O₈⁻, B₅H₄O₁₀⁻, [BF₄]⁻, [BCl₄]⁻, [B(C₆H₅)₄]⁻, [PF₆]⁻, [SbF₆]⁻' [AsF₆]⁻' [AlCl₄]⁻, [AlBr₄]⁻, [ZnCl₃]⁻, dichlorocuprates (I) and (II), CO₃²⁻, HCO₃⁻, F⁻, (R'-COO)', R'₃SiO⁻, (R'-SO₃)⁻ and [(R'-SO₂)₂N]⁻, where R' is alkyl, cycloalkyl or aryl.

8. The process according to any of the preceding claims, wherein the reaction in step a) is carried out at a temperature of at least 40°C, preferably at least 80°C, in particular in the range from 100 to 220°C.

9. The process according to any of the preceding claims, wherein in step a), the amine compound is firstly brought into contact with the dialkyl sulfate or the trialkyl phosphate at a temperature of not more than 30°C and the resulting mixture is subsequently heated to a temperature of at least 40°C.

10. The process according to any of the preceding claims, wherein the reaction in step a) is carried out in an organic solvent, in water or in a mixture thereof.

11. The process according to claim 10, wherein the solvent comprises at least 30% by volume of water.

12. The process according to any of the preceding claims, wherein the reaction in step a) is carried out in the presence of an inert gas.

13. The process according to any of the preceding claims, wherein the process steps a) and b) are carried out in the absence of halide ions.

14. The process according to any of the preceding claims, wherein the anion exchange in step b) is effected by transprotonation, reaction with a metal salt, ion exchange chromatography, electrolytically or by means of a combination thereof.

15. The process according to claim 14, wherein the reaction with the metal salt is carried out in a solvent from which a metal sulfate formed from the metal of the metal salt and the sulfate anion crystallizes.

16. The process as defined in any of claims 1 to 15 for preparing high-purity aqueous tetraalkylammonium hydroxide solutions, wherein, in each case in water and in the absence of compounds and of materials which comprise or liberate halide ions,
a) a trialkylamine compound is reacted with a dialkyl sulfate or trialkyl phosphate to give a high-purity aqueous tetraalkylammonium salt solution and
b) the tetraalkylammonium salt solution obtained in step a) is subjected to an electrolytic anion exchange or ion exchange chromatography to replace the anions by hydroxide ions.

## Revendications

1. Procédé pour la préparation d'un composé d'ammonium quaternaire, dans lequel
a) on transforme un composé d'amine, qui contient au moins un atome d'azote hybridé en sp³, avec un sulfate de dialkyle ou un phosphate de trialkyle avec obtention d'un composé d'ammonium quaternaire qui présente au moins partiellement des anions polyvalents et
b) on soumet le composé d'ammonium quaternaire obtenu dans l'étape a) à un échange anionique.

2. Procédé selon la revendication 1, dans lequel on transforme dans l'étape a) le composé d'amine avec un sulfate de dialkyle en utilisant les deux groupes alkyle du sulfate de dialkyle, un composé d'ammonium quaternaire présentant des anions sulfate étant obtenu.

3. Procédé selon la revendication 1, dans lequel on transforme dans l'étape a) le composé d'amine avec un phosphate de trialkyle en utilisant au moins deux des groupes alkyle du phosphate de trialkyle, un composé d'ammonium quaternaire présentant des anions phosphate et/ou des anions monoalkylphosphate étant obtenu.

4. Procédé selon l'une quelconque des revendications précédentes, le composé d'amine utilisé dans l'étape a) étant choisi parmi les composés de formule NR¹R²R³, dans laquelle R¹, R² et R³ sont choisis, indépendamment les uns des autres, parmi hydrogène, alkyle, cycloalkyle, hétérocycloalkyle, aryle et hétéroaryle, au moins deux des radicaux R¹, R² et R³ pouvant également être, ensemble avec l'atome N auquel ils sont liés, une partie d'un composé polycyclique.

5. Procédé selon l'une quelconque des revendications précédentes, le composé d'amine utilisé dans l'étape a) portant au moins un autre substituant qui est choisi parmi les groupes hydroxyle, les groupes mercapto, les groupes ester, les groupes thioester et leurs combinaisons.

6. Procédé selon la revendication 5, le composé d'amine utilisé dans l'étape a) étant choisi parmi les aminoalcools, les aminothiols et leurs esters avec des acides inorganiques, en particulier l'O-phosphate de choline, l'O-sulfate de choline et l'O-phosphatidylcholine.

7. Procédé selon l'une quelconque des revendications précédentes, le composé ionique obtenu comprenant au moins un anion Xⁿ⁻' n représentant un nombre entier correspondant à la valence de l'anion, qui est choisi parmi OH⁻' HSO₄⁻' NO₂⁻' NO₃⁻', CN⁻' OCN⁻' NCO⁻' SCN⁻' NCS⁻¹ PO₄³⁻' HPO₄²⁻' (H₂PO₄⁻), H₂PO₃⁻' HPO₃²⁻' BO₃³⁻' (BO₂)₃³⁻' B₅O₆⁻' B₅O₈⁻' B₅H₄O₁₀⁻' [BF₄]⁻' [BCl₄]⁻' [B(C₆H₅)₄]⁻' [PF₆]⁻' [SbF₆]⁻' [AsF₆]⁻' [AlCl₄]⁻' [AlBr₄]⁻' [ZnCl₃]⁻' les dichlorocuprates (I) et (II), CO₃²⁻' HCO₃⁻' F⁻' (R'-COO)⁻' R'₃SiO⁻' (R'-SO₃)⁻ et [(R'-SO₂)₂N]⁻' dans lesquels R' représente alkyle, cycloalkyle ou aryle.

8. Procédé selon l'une quelconque des revendications précédentes, la transformation dans l'étape a) étant réalisée à une température d'au moins 40°C, de préférence d'au moins 80°C, en particulier dans la plage de 100 à 220°C.

9. Procédé selon l'une quelconque des revendications précédentes, le composé d'amine étant d'abord mis en contact, dans l'étape a), avec le sulfate de dialkyle ou le phosphate de trialkyle à une température d'au plus 30°C et le mélange résultant étant ensuite chauffé à une température d'au moins 40°C.

10. Procédé selon l'une quelconque des revendications précédentes, la transformation dans l'étape a) étant réalisée dans un solvant organique, dans l'eau ou dans un mélange de ceux-ci.

11. Procédé selon la revendication 10, le solvant comprenant au moins 30% en volume d'eau.

12. Procédé selon l'une quelconque des revendications précédentes, la transformation dans l'étape a) ayant lieu en présence d'un gaz inerte.

13. Procédé selon l'une quelconque des revendications précédentes, les étapes de procédé a) et b) étant réalisées en l'absence d'ions halogénure.

14. Procédé selon l'une quelconque des revendications précédentes, le remplacement de l'anion dans l'étape b) étant réalisé par transprotonation, transformation avec un sel métallique, chromatographie par échange d'ions, électrolyse ou une combinaison de celles-ci.

15. Procédé selon la revendication 14, la transformation avec le sel métallique étant réalisée dans un solvant, à partir duquel un sulfate de métal formé à partir du métal du sel métallique et de l'anion sulfate se sépare par cristallisation.

16. Procédé tel que défini dans l'une quelconque des revendications 1 à 15 pour la préparation de solutions aqueuses d'hydroxyde de tétraalkylammonium de haute pureté dans lequel, à chaque fois dans l'eau et en excluant des composés et des matériaux qui contiennent des ions halogénure ou qui les libèrent,
a) on transforme un composé de trialkylamine avec un sulfate de dialkyle ou un phosphate de trialkyle, avec obtention d'une solution aqueuse de sel de tétraalkylammonium de haute pureté et
b) on soumet la solution de sel de tétraalkylammonium obtenue dans l'étape a) à un échange anionique électrolytique ou une chromatographie par échange d'ions pour le remplacement des anions par des ions d'hydroxyde.
